(19) **Europäisches Patentamt**

**European Patent Office**

**Office européen des brevets**

(11) Publication number: **0 438 747 A1**

(12) # EUROPEAN PATENT APPLICATION

(21) Application number: **90124974.8**

(22) Date of filing: **20.12.90**

(51) Int. Cl.⁵: **A61K 37/02, A61K 9/14, A61K 47/26, A61K 47/36**

(30) Priority: **22.12.89 JP 333748/89**

(43) Date of publication of application:
**31.07.91 Bulletin 91/31**

(84) Designated Contracting States:
**AT BE CH DE DK ES FR GB GR IT LI LU NL SE**

(71) Applicant: **SHIONOGI SEIYAKU KABUSHIKI KAISHA trading under the name of SHIONOGI & CO. LTD.**
**1-8, Doshomachi 3-chome Chuo-ku Osaka 541(JP)**

(72) Inventor: **Tsukada, Takayuki**
**A-716, 2-136-3 Kasugaoka**
**Itami-shi, Hyogo(JP)**
Inventor: **Shima, Kazuhiro**
**C-7-406, 3-1 Shinsenrinishimachi**
**Toyonaka-shi, Osaka(JP)**
Inventor: **Takagishi, Yasushi**
**27-1, Shiomi-cho**
**Ashiya-shi, Hyogo(JP)**

(74) Representative: **Vossius & Partner**
**Siebertstrasse 4 P.O. Box 86 07 67**
**W-8000 München 86(DE)**

(54) **Stabilized composition of glycopeptide antibiotics.**

(57) The stabilized freeze-dry composition of glycopeptide antibiotics comprising one or more saccharides at 0.05 parts by weight or more. In this invention, glycopeptide antibiotics means orenticins A to D, chloroorienticins A to E, and vancomycin.

## STABILIZED COMPOSITION OF GLYCOPEPTIDE ANTIBIOTICS

The present invention relates to stable compositions of glycopeptide antibiotics. Furthermore, it relates to stable freeze-dried compositions containing mineral acid addition salts of glycopeptide antibiotics and one ore more saccharides.

As to glycopeptide antibiotics, their mineral acid addition salts have been often used in an injectable form of powders or in freeze-dried ones, which are dissoloved just before administration. However, the mineral acid addition salts of glycopeptide antibiotics turn light red or light yellowish red during a long shelf-life and consequently undergo chemical decomposition. The degree of their discoloration and decomposition is developed in proportion to the residual water content in the mineral acid salt and the term and temperature of the storage. Further, the disoloration is influenced largely by oxygen in the air. Such a chemical change causes to spoil the antibacterial activity and safety, the fact of which causes a problem.

To solve the above mentioned problem, the following methods were executed: (1) reducing the water content of the product in vials by drying; (2) replacing the air in vials by inert gas such as nitrogen gas; and (3) storing the product at a lower temperature.

The present invention relates to a stabilized freeze-dry composition comprising 0.05 parts by weight or more of one or more saccharides.

The present invention relates to stabilized compositions of glycopeptide antibiotics, which means orienticins A to D which are disclosed in Tsuji et al [N. Tsuji et al., The Journal of Antibiotics, Vol.41, No. 6, 819-822 (1988); ibid Vol.41, No.10, 1506-1510 (1988)], chloroorienticins A to E, and vancomycin. However, the free bases of the glycopeptide antibiotics are hardly soluble in water and, therefore, this form is not adequate to be applied as bulk powder for injection as it is. Therefore, the free bases of the glycopeptide antibiotics are changed into water soluble salts, and the obtained salts are usually subjected to be applied for the injection. Water-soluble salts mean acid addition salts with mineral acids, such as the hydrochloride-, sulfate-, and nitrate-salts, and the hydrochloride salt is preferred. 0.5 or more moles of the hydrochloride for the addition salt are necessary to solubilize 1 mole of the glycopeptide antibiotics. However, the theoretical ratio is 1:1.

Saccharides are the water-soluble ones including mono-saccharides, di-saccharides, sugar-alcohols, and poly-saccharides. Namely mono-saccharides include glucose, fructose, mannose, sorbose, and xylose.

Di-saccharides include maltose, saccharose, and lactose.

Sugar-alcohols include mannitol, sorbitol, inositol, and xylitol.

Polysaccharides include dextran, pluran, amylose, dextrin, cyclodextrin, and the derivatives thereof.

Saccharides have optical isomers and those which are constituted by monosaccharides in different bindings. All of them may be used for this invention.

Physiologically acceptable substances such as sodium chloride, phosphoric acid, citric acid, tartaric acid, succinic acid, or the salts thereof can be further added thereto to elevate the solubility and quickness in the dissolution into an aqueous medium, and to adjust pH values and osmotic pressure when reconstituted.

For accomplishing the compositon of the present invention, the quantity of the saccharides used should not be limited, but preferably it may be 0.05 parts by weight or more to the mineral acid addition salt of glycopeptide antibiotics, more preferably over 0.15 parts by weight, especially over 0.3 parts by weight.

The compositions of the present invention can be prepared by freeze-drying methods. For example, the mineral acid addition salts of the glycopeptide antibiotics and one or more saccharides are dissolved in an aqueous medium and the obtained solution is filterd aseptically with a membrane filter. Successively, the resulting filtrate is subjected to freeze-drying to obtain the composition of the present invention.

The freeze-drying method includes a bulk-dry method using a tray and a method where a the sterile solution is dissolved into vials or ampules and then freeze-dried. The latter method is usually adopted, and the thus prepared vials or ampules are charged with inactive gas, such as nitrogen, argon, and carbon dioxide, and then sealed.

The invention is illustrated by the following examples, which are not to be considered as limiting the scope.

Experiment 1

The following experiment proves that a saccharide prevented the mineral acid addition salt of glycopeptide antibiotics from discoloration and decomposition.

Chloroorienticin B • mono-hydrochloride (100 mg) is dissolved in water for injection (4ml) together with 20 mg of the saccharides or with 50 mg of dextran 70 as stabilizer as illustrated in Table 1. The mixture is filtered aseptically and the filtrate is filled into 3 ml-vials by 1 ml each and then frozen immediately and dried in vacuo. After the samples are dried, the vials are filled with nitrogen gas and sealed to give test compositions. As a control, each vial is opened once to replace the nitrogen gas with air, and then sealed again without addition of the stabilizing agent to give the pharmaceutical preparations. The test compositions and reference compositions are subjected to a one-month accelerated stability test at 50 °C, to observe the change in appearance (color) and to measure the degree of the chemical decomposition. The results are also shown in Table 1.

Table 1

| Atmosphere | | Nitrogen | | Air | |
|---|---|---|---|---|---|
| Stabilizing Agent | Content (mg) | Change in appearance | Remaining rate (%) | Change in appearance | Remaining rate (%) |
| None (Control) | 0 | + 1R | 89.5 | + 2R | 90.0 |
| Mannitol | 20 | – | 95.8 | – | 96.5 |
| Glucose | 20 | ± Y | 93.9 | ± Y | 94.4 |
| Maltose | 20 | – | 95.7 | – | 95.8 |
| Dextran-70 | .50 | + 1R | 95.6 | + 1R | 95.5 |

"Content (mg)" of stabilizing agent: shown by weight added to 100 mg of chloroorienticin B · hydrochloride.

"Change in appearance": observation of the coloration of the dried composition in the transparent glass vials with the naked eye.

This evaluation was carried out according to the following criteria:

R : light reddish
Y : yellowish
– : without coloration just after the preparation
± : very slight coloration
+1 : slight coloration
+2 : apparent coloration

"Remaining rate (%)": It was measured with HPLC (high performance liquid chromatography) under following conditions: It is shown as the rate (%) of the drug compared with the drug contents just after the preparation.

High pressure pump : Shimadzu LC-6A Pump
Detector : Shimadzu SPD-6A
Detective wavelength : 220 nm
Data processor : Shimadzu C-R3A
Column : Nakarai Chemical Cosmosil 5ph; 4.6mmID×150mmL
Eluting solution : 0.05 M pH3.5 phosphate buffer/acetonitrile (91/9)
Flow rate : 1.0 ml/min.
Retention time : about 15 min.
The medium of test solution : purified water

EP 0 438 747 A1

It is self-explanatory from Table 1 that the added saccharides, which were subjected to the test, significantly prevented chloroorienticin B · hydrochloride from discoloration, even if in the presence of the air. Furthermore, the effect for the prevention of chemical decomposition were recognized as being independent from the kind of the gas in the vials, i.e. whether it is inactive or not.

Example 1

Chloroorienticin B · hydrochloride (0.25 g) and mannitol (0.125 g) as a stabilizing agent are dissolved in 2.5 ml of water for injection. The solution (55 ml) is aseptically filtered with a membrane filter (0.22 $\mu$m), and filled into 14 ml-vials by 2.5 ml each. According to a conventional manner of the freeze-drying method, the vial is cooled down to -40 °C, retained for 1 hour, and subjected to sublimation at a temperature under -20 °C at a pressure below 0.1 millibar to remove the water. After drying, the vials are filled with nitrogen gas and sealed to give the freeze-dried preparations for injection.

Example 2

In the same manner as in Example 1, except that 0.0375 g of mannitol is used as stabilizer, a solution (55 ml) is prepared and dispensed to 14 ml-vials by 2.5 ml each after filtration. The vials are subjected to freeze-drying to give the freeze-dried preparations for injection.

Example 3

In the same manner as in Example 1, except that 0.075 g of mannitol is used as stabilizer, a solution (55 ml) is prepared and dispensed to 14 ml-vials by 2.5 ml each after filtration. The vials are subjected to freeze-drying to give the freeze-dried preparations for injection.

Example 4

In the same manner as in Example 1, except that 0.125 g of mannitol is used as stabilizer, a solution (55 ml) is prepared and dispensed to 14 ml-vials by 2.5 ml each after filtration. The vials are subjected to freeze-drying to give the freeze-dried preparations for injection.

Example 5

Chloroorienticin B · hydrochloride (0.25 g) and maltose (0.0375 g) as a stabilizing agent are dissolved in 2.5 ml of water for injection. The solution (55 ml) is filtered in the same manner as Example 1, and filled to 14 ml-vials by 2.5 ml each. The vials are subjected to freeze- drying to give the freeze-dried preparations for injection.

Example 6

In the same manner as in Example 5, except that 0.075 g of maltose is used as stabilizer, a solution (55 ml) is prepared and dispensed to 14 ml-vials by 2.5 ml each after filtration. The vials are subjected to freeze-drying to give the freeze-dried preparations for injection.

Example 7

Chloroorienticin B · hydrochloride (0.25 g) and mannitol (0.075 g) as a stabilizing agent, and citric acid (0.0063 g) as a pH regulator are dissolved in 2.5 ml of water for injection. The solution (55 ml) is filtered in the same manner as Example 1, and filled to 14 ml-vials by 2.5 ml each. The vials are subjected to freeze-drying to give the freeze-dried preparations for injection.

Example 8

In the same manner as in Example 7, except that 0.0125 g of citric acid is used as a pH regulator, a solution (55 ml) is prepared and dispensed to 14 ml-vials by 2.5 ml each after filtration. The vials are subjected to freeze-drying to give the freeze-dried preparations for injection.

Example 9

Chloroorienticin B • hydrochloride (0.25 g), mannitol (0.075 g) as a stabilizing agent, and sodium chloride (0.0063 g) as an osmotic pressure regulator are dissolved in 2.5 ml of water for injection. The solution (55 ml) is filtered in the same manner as Example 1, and filled to 14 ml-vials by 2.5 ml each. The vials are subjected to freeze-drying to give the freeze-dried preparations for injection.

Example 10

Chloroorienticin B • hydrochloride (0.25 g) and sorbitol (0.075 g) as a stabilizing agent are dissolved in 2.5 ml of water for injection. The solution (40 ml) is filtered in the same manner as Example 1, and filled to 14 ml-vials by 2.5 ml each. The vials are subjected to freeze-drying to give the freeze-dried preparations for injection.

Example 11

Chloroorienticin B • hydrochloride (0.25 g) and xylitol (0.075 g) as a stabilizing agent are dissolved in 2.5 ml of water for injection. The solution (40 ml) is filtered in the same manner as Example 1, and filled to 14 ml-vials by 2.5 ml each. The vials are subjected to freeze-drying to give the freeze-dried preparations for injection.

Exmaple 12

Chloroorienticin B • hydrochloride (0.25 g) and inositol (0.075 g) as a stabilizing agent are dissolved in 2.5 ml of water for injection. The solution (40 ml) is filtered in the same manner as Example 1, and filled to 14 ml-vials by 2.5 ml each. The vials are subjected to freeze-drying, to give the freeze-dried preparations for injection.

Example 13

Vancomycin • hydrochloride (0.025 g) and mannitol (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with nitrogen gas and sealed to give the freeze-dried preparations for injection.

Example 14

Vancomycin • hydrochloride (0.025 g) and mannitol (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with air and sealed to give the freeze-dried preparations for injection.

Example 15

Vancomycin • hydrochloride (0.025 g) and glucose (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with nitrogen gas and sealed to give the freeze-dried preparations for injection.

Example 16

Vancomycin • hydrochloride (0.025 g) and glucose (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with air and sealed to give the freeze-dried preparations for injection.

Example 17

Vancomycin • hydrochloride (0.025 g) and maltose (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with nitrogen gas and sealed to give the freeze-dried preparations for injection.

Example 18

Vancomycin • hydrochloride (0.025 g) and maltose (0.005 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with air and sealed to give the freeze-dried preparations for injection.

Example 19

Vancomycin • hydrochloride (0.025 g) and dextran 70 (0.125 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with nitrogen gas and sealed to give the freeze-dried preparations for injection.

Example 20

Vancomycin • hydrochloride (0.025 g) and dextran 70 (0.125 g) as a stabilizing agent are dissolved in 1 ml of water for injection. The solution (20 ml) is filtered in the same manner as Example 1, and filled to 1 ml-vials by 1 ml each. The vials are subjected to freeze-drying, filled with air and sealed to give the freeze-dried preparations for injection.

Experiment 2

The freeze-dried preparations obtained in Example 1-6, which contain mannitol or maltose, and control preparations without additive agent are subjected to an accelerated stability test. Each preparation is preserved at 50 °C subsequently the change in appearance (color), remaining rate, and the discoloration degree at liquid form are measured. The change in appearance and the remaining rate are measured in the same manner as Example 1. The discoloration in the liquid form is measured by the following method: The vials are dissolved in 2.5 ml of water for injection and the discoloration of the solution is immediately measured as the absorbance with a wave length of 465 nm and optical path length of 1 cm.
The results are shown in Table 2.

Table 2
Accelerated stability test at 50°C

| Example No. | Stabilizing agent | Content (mg) | 50°C-0.5M | | | 50°C-1M | | |
|---|---|---|---|---|---|---|---|---|
| | | | Remaining rate (%) | Change in appearance | Coloration | Remaining rate (%) | Change in appearance | Coloration |
| Control | None | 0 | 95.5 | +1 RY | 0.029 | 92.9 | +1~+2RY | 0.070 |
| 1 | Mannitol | 5 | 97.0 | ±~+1RY | 0.020 | 96.0 | +1~+2RY | 0.048 |
| 2 | Mannitol | 15 | 98.5 | ± | 0.015 | 98.0 | +1 RY | 0.029 |
| 3 | Mannitol | 30 | 98.8 | - | 0.010 | 98.7 | - | 0.022 |
| 4 | Mannitol | 50 | 99.3 | - | 0.010 | 99.3 | - | 0.021 |
| 5 | Maltose | 15 | 96.7 | +1 RY | 0.018 | 96.1 | +1~+2RY | 0.042 |
| 6 | Maltose | 30 | 97.7 | ±~+1RY | 0.028 | 97.4 | +1 RY | 0.033 |

Comparison of the preparations which contain mannitol or maltose with the control preparation without additive agent, results in the observation that the former preparations suppressed and prevented significantly the decreasing of the drug content and the discoloration. Further, these effects of the suppression or prevention show more clearly the more mannitol or maltose is added.

Experiment 3

Freeze-dried preparations containing mannitol and citric acid as a pH regulator, mannitol and sodium chloride as an osmotic pressure regulator, sorbitol, xylitol, or inositol instead of mannitol, (which are obtained in Example 7-12), and the oontrol preparation A without additives, and the control preparation B containing only mannitol as a stabilizer were subjected to the accelerated stability test. The test and measurement were performed in the same manner as Experiment 2. The results are shown in Table 3.

Table 3

Accelerated stability test at 50°C

| Example No. | Stabilizing agent A (mg) | Stabilizing agent B (mg) | 50°C-0.5M | | | 50°C-1M | | |
|---|---|---|---|---|---|---|---|---|
| | | | Remaining rate (%) | Change in appearance | Colora-tion | Remaining rate (%) | Change in appearance | Colora-tion |
| Control A | None | None | 95.8 | ±-+1RY | 0.029 | 93.8 | +1 RY | 0.058 |
| Control B | Mannitol 30 | None | 99.8 | - | 0.010 | 99.8 | - | 0.016 |
| 7 | Mannitol 30 | citric acid 2.5 | 99.4 | - | 0.012 | 99.5 | - | 0.011 |
| 8 | Mannitol 30 | citric acid 5.0 | 99.0 | - | 0.013 | 98.9 | - | 0.011 |
| 9 | Mannitol 30 | sodium chloride 2.5 | 99.6 | - | 0.013 | 99.9 | - | 0.023 |
| 10 | Sorbitol 30 | None | 99.5 | - | 0.011 | 99.3 | - | 0.013 |
| 11 | Xylitol 30 | None | 99.1 | - | 0.013 | 98.7 | - | 0.015 |
| 12 | Inositol 30 | None | 98.9 | - | 0.013 | 98.5 | - | 0.016 |

EP 0 438 747 A1

The preparations containing citric acid or sodium chloride in addition to mannitol are stabilized to a significantly higher degree than the control preparation A, and have the same effect as the control preparation B. Further, these effects are not reduced by the addition of citric acid or sodium chloride. The preparations containing sugar alcohol in place of mannitol as a stabilizing agent suppressed and prevented more significantly the decreasing of the drug content, the change in appearance and the discoloration as compared with the control preparation without these agents. The above effects of the sugar alcohol are inferior to mannitol.

Experiment 4

The preparations obtained in Examples 13-20, which contain mannitol, glucose, maltose, or dextran 70 respectively are filled with nitrogen gas or air, preserved at 50 °C, and subjected to the stability test. The change in appearance and the remaining rate, are both measured in the same manner as Example 1. The results are shown in Table 4.

Table 4

| Atmosphere | | Nitrogen | | | Air | | |
|---|---|---|---|---|---|---|---|
| Stabilizing agent | Content (mg) | Ex. No. | Change in appearance | Remaining rate (%) | Ex. No. | Change in appearance | Remaining rate (%) |
| None (Control) | 0 | − | + 1R | 91.3 | − | + 2R | 91.1 |
| Mannitol | 20 | 13 | ± R | 98.0 | 14 | ± R | 97.8 |
| Glucose | 20 | 15 | ± Y | 96.5 | 16 | ± YR | 94.4 |
| Maltose | 20 | 17 | ± R | 98.5 | 18 | + 1R | 98.1 |
| Dextran-70 | 50 | 19 | + 1R | 95.4 | 20 | + 1R | 95.5 |

"Content (mg)" of the stabilizing agent: shown by weight added to 100 mg of vancomycin · hydrochloride.
The measurement of the "remaining rate (%)" was performed in the same manner as Experiment 1
except for the following conditions:

Eluting solution : 0.05 M pH3.5 phosphate buffer/acetonitorile (92.5/7.5)
Retention time : about 11 min.

EP 0 438 747 A1

EP 0 438 747 A1

It is self-explanatory from Table 4 suppressed significantly the decreasing of the drug content irrespective of whether the filled gas was either air or nitrogen, as compared with the control preparation without stabilizing agent, and that the preparations containing the stabilizing agent further suppressed the discoloration in the presence of air.

## Claims

1. A stabilized freeze-dried composition of a glycopeptide antibiotic, comprising at least 0.05 weight-parts of one or more of water-soluble saccharides.

2. The composition as claimed in Claim 1, wherein said glycopeptide antibiotic is the salt of chloroorienticin B or vancomycin with a mineral acid.

3. The composition as claimed in Claim 2, wherein said mineral acid is hydrochloric acid.

4. The composition as claimed in Claim 1, wherein said saccharide is a mono-saccharide, di-saccharide, sugar-alcohol, or a water-soluble poly-saccharide, or a mixture thereof.

5. The composition as claimed in Claim 4, wherein said mono-saccharide is glucose, fructose, mannose, sorbose, or xylose, or a mixture thereof.

6. The composition as claimed in Claim 4, wherein said di-saccharide is maltose, saccharose, or lactose, or a mixture thereof.

7. The composition as claimed in Claim 4, wherein said sugar-alcohol is mannitol, sorbitol, inositol, or xylitol, or a mixture thereof.

8. The composition as claimed in Claim 4, wherein said poly-saccharide is dextran.

9. A process for the preparation of a stabilized freeze-dried composition of a glycopeptide antibiotic wherein at least 0.05 weight-parts of one or more of water-soluble saccharides are added to an aqueous acid addition salt solution of the glycopeptide antibiotic and subsequently freeze-drying the resulting solution.

10. Use of at least 0.05 weight-parts of one or more water-soluble saccharides for the preparation of a stabilized freeze-dried composition of a glycopeptide antibiotic.

## CLAIMS FOR THE FOLLOWING CONTRACTING STATES: GR AND ES

1. A process for the preparation of a stabilized freeze-dried composition of a glycopeptide antibiotic wherein at least 0.05 weight-parts of one or more of water-soluble saccharides are added to an aqueous acid addition salt solution of the glycopeptide antibiotic, and subsequently freeze-drying the resulting solution.

2. The process as claimed in Claim 1, wherein said glycopeptide antibiotic is the salt of chloroorienticin B or vancomycin with a mineral acid.

3. The process as claimed in Claim 2, wherein said mineral acid is hydrochloric acid.

4. The process as claimed in Claim 1, wherein said saccharide is a mono-saccharide, di-saccharide, sugar-alcohol, or a water-soluble poly-saccharide, or a mixture thereof.

5. The process as claimed in Claim 4, wherein said mono-saccharide is glucose, fructose, mannose, sorbose, or xylose, or a mixture thereof.

6. The process as claimed in Claim 4, wherein said di-saccharide is maltose, saccharose, or lactose, or a mixture thereof.

13

7. The process as claimed in Claim 4, wherein said sugar-alcohol is mannitol, sorbitol, inositol, or xylitol, or a mixture thereof.

8. The process as claimed in Claim 4, wherein said poly-saccharide is dextran.

9. Use of at least 0.05 weight-parts of one or more water-soluble saccharides for the preparation of a stabilized freeze-dried composition of a glycopeptide antibiotic.

European
Patent Office

**EUROPEAN SEARCH
REPORT**

Application Number

**EP 90 12 4974**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 078 451 (BEHRINGWERKE AG)<br>* Abstract; page 1, lines 1-12; page 4, lines 3-11,29-34; claims 1,3,5 * | 1,4,6,10 | A 61 K 37/02<br>A 61 K 9/14<br>A 61 K 47/26<br>A 61 K 47/36 |
| X | FR-A-2 396 019 (LABORATOIRES CASSENNE)<br>* Page 1, lines 19-25; page 5, lines 13-34; example 4; claim 12 * | 1,4,7,10 | |
| Y | | 2,3,9 | |
| Y | EP-A-0 318 146 (ELI LILLY CO.)<br>* Abstract; page 2, lines 1-11; example 1; claim 6 * | 2,3,9 | |
| Y | FR-A-2 101 041 (LABORATOIRES CASSENNE)<br>* Page 1, lines 6-8; page 4, lines 6-8; page 13, line 34 - page 14, line 2 * | 1-10 | |
| D,Y | THE JOURNAL OF ANTIBIOTICS, vol. 41, no. 10, October 1988, pages 1506-1510; N. TSUJI et al.: "New glycopeptide antibiotics: II. The isolation and structures of chloroorien-ticins"<br>* Whole document * | 1-10 | |
| Y | US-A-4 102 999 (UMEZAWA et al.)<br>* Abstract; column 1, lines 10-16; column 2, line 7 - column 3, line 19 * | 1-10 | TECHNICAL FIELDS SEARCHED (Int. Cl.5)<br><br>A 61 K |
| A | EP-A-0 306 312 (YAMANOUCHI PHARMACEUTICAL)<br>* Abstract; page 2, lines 18-32; claims 1,4,5 * | 1-10 | |

The present search report has been drawn up for all claims

| Place of search | Date of completion of search | Examiner |
|---|---|---|
| The Hague | 11 April 91 | HOFF P.J.L. |

CATEGORY OF CITED DOCUMENTS

X : particularly relevant if taken alone
Y : particularly relevant if combined with another
document of the same category
A : technological background
O : non-written disclosure
P : intermediate document
T : theory or principle underlying the invention

E : earlier patent document, but published on, or after
the filing date
D : document cited in the application
L : document cited for other reasons

                                                 

& : member of the same patent family, corresponding
document

ANNEX TO THE EUROPEAN SEARCH REPORT
ON EUROPEAN PATENT APPLICATION NO.

EP 90 12 4974

This annex lists the patent family members relating to the patent documents cited in the above-mentioned European search report.
The members are as contained in the European Patent Office EDP file on 06/05/91
The European Patent Office is in no way liable for these particulars which are merely given for the purpose of information.

| Patent document cited in search report | Publication date | Patent family member(s) | | Publication date |
|---|---|---|---|---|
| EP-A- 0078451 | 11-05-83 | DE-A- | 3141894 | 05-05-83 |
| | | AU-B- | 549733 | 06-02-86 |
| | | AU-A- | 8967482 | 28-04-83 |
| | | CA-A- | 1191771 | 13-08-85 |
| | | JP-A- | 58078599 | 12-05-83 |
| | | US-A- | 4510126 | 09-04-85 |
| FR-A- 2396019 | 26-01-79 | None | | |
| EP-A- 0318146 | 31-05-89 | US-A- | 4885275 | 05-12-89 |
| | | AU-A- | 2369688 | 08-06-89 |
| | | JP-A- | 1146825 | 08-06-89 |
| FR-A- 2101041 | 31-03-72 | BE-A- | 771042 | 07-02-72 |
| | | DE-A- | 2140353 | 17-02-72 |
| | | GB-A- | 1357564 | 26-06-74 |
| | | NL-A- | 7111115 | 15-02-72 |
| | | SE-B- | 388125 | 27-09-76 |
| US-A- 4102999 | 25-07-78 | JP-C- | 1021079 | 25-11-80 |
| | | JP-A- | 51095118 | 20-08-76 |
| | | JP-B- | 55008967 | 07-03-80 |
| EP-A- 0306312 | 08-03-89 | JP-A- | 1156925 | 20-06-89 |

EPO FORM P0459